**Europäisches Patentamt**

**European Patent Office**

**Office européen des brevets**

(11) Veröffentlichungsnummer: **0 323 800**

**A1**

## (12) EUROPÄISCHE PATENTANMELDUNG

(21) Anmeldenummer: **88730284.2**

(22) Anmeldetag: **29.12.88**

(51) Int. Cl.4: **A61F 2/30**

(30) Priorität: **05.01.88 DE 3800259**

(43) Veröffentlichungstag der Anmeldung:
**12.07.89 Patentblatt 89/28**

(84) Benannte Vertragsstaaten:
**DE ES FR GB**

(71) Anmelder: **MECRON medizinische Produkte GmbH**
**Nunsdorfer Ring 23-29**
**D-1000 Berlin 48(DE)**

(72) Erfinder: **Meffert, Oskar, Prof. Dr. med.**
**Krankenhaus Oststadt Podbielskistrasse 380**
**D-3000 Hannover 51(DE)**

(74) Vertreter: **Christiansen, Henning, Dipl.-Ing.**
**Patentanwalt CHRISTIANSEN Pacelliallee 43/45**
**D-1000 Berlin 33(DE)**

(54) **Implantierbare Prothese.**

(57) Implantierbare Prothese mit einem Prothesenkopf und einem Prothesenschaft, wobei zumindest der Prothesenschaft (2) eine Umhüllung (3) aus biokompatiblem, elastischem, membranartigem Kunststoff aufweist.

EP 0 323 800 A1

## Implantierbare Prothese

Die Erfindung betrifft eine implantierbare Prothese mit einem Prothesenkopf und einem Prothesenschaft.

Es ist bekannt, Prothesen beispielsweise zum Ersatz eines Hüftgelenks in den entsprechend vorbereiteten Knochen ei nes Patienten unter Verwendung eines Knochenzements zu implantieren, so daß nach dem Erhärten des Knochenzements eine feste Verbindung zwischen dem Prothesenschaft und der Markhöhle des betreffenden Knochen gegeben ist. Da der Knochenzement aufgrund seiner Eigenschaften nach längerer Zeit spröde wird, kommt es wegen der wechselnden Belastungen der implantierten Prothese zu Auslockerungen, die dem Patienten Schmerzen bereiten und den Knochen bzw. das Gelenk zerstören könnten, so daß eine Reimplantation erforderlich wird. Aus diesem Grunde werden häufig zementfreie Implantationen bevorzugt, bei denen profilierte Prothesen verwendet werden, die kraft- und/oder formflüssig in dem betreffenden Knochen des Patienten verankert werden. Eine solche Verankerung schließt aber in der Regel eine Reimplantation aus, da das Entfernen einer profilierten Prothese aus der Markhöhle des betreffenden Knochen in vielen Fällen nur mit einer Zerstörung der Knochenstruktur möglich ist.

Der Erfindung liegt die Aufgabe zugrunde, eine implantierbare Prothese der eingangs genannten Gattung zu schaffen, die kraftflüssig in dem Knochen eines Patienten verankert wird und eine verbesserte Langzeitverbindung zwischen Knochen und Prothesen auch bei stark wechselnder Beanspruchung der Prothese sicherstellt. Diese Aufgabe wird mit dem kennzeichnenden Merkmal des Anspruches 1 gelöst.

Die erfindungsgemäße Lösung gewährleistet eine verbesserte Verbindung zwischen der Prothese und dem Knochen, da die Umhüllung aus einem biokompatiblen, elastischen, membranartigen Kunststoff einen felxiblen Puffer bildet, der zum einen die wechselnden Beanspruchungen der implantierten Prothese aufnimmt und zum anderen aufgrund seiner Biokompatibilität und seines membranartigen Verhaltens mit der damit verbundenen Wasserdampfdurchlässigkeit zu keinen Reaktionen des die implantierte Prothese umgebenden Knochengewebes führt, und auch die Gefahr einer Auslockerung der implantierten Prothese erhöhen würde.

Die so entstehende elastische Zwischenschicht ist insbesondere in der Lage, Kraftspitzen abzubauen und damit die Knochenstruktur schädigenden lokalen Überbeanspruchungen zu vermeiden. Auch sind die Auswirkungen von schädlichen Mikrobewegungen unterbunden.

Eine vorteilhafte Weiterbildung der erfindungsgemäßen Lösung ist dadurch gekennzeichnet, daß die Umhüllung aus einem Polytretrafluoräthylen-Material, insbesondere einem unter der Bezeichnung "Gore-Tex" von der Firma W. L. Gore and Associates, Flagstaff, Arizona, USA hergestellten Polytretrafluoräthylen (PTFE)-Material besteht. Dieses Material findet aufgrund seiner Wasser- und Winddichtigkeit sowie seiner Atmungsaktivität und membranartigen Funktion in der Bekleidungsindustrie sowie in der Gefäßchirugie Verwendung, wo es als Transplantat für Blutgefäße eingesetzt wird. Das membranartige Verhalten dieses Materials in Verbindung mit seiner Elastizität verhindert ein Verspröden, so daß im Gegensatz zur Verwendung eines Knochenzements zur Verankerung einer implantierbaren Prothese in der Markhöhle eines Knochens keine Auslockerungen nach längerer Zeit zu befürchten sind.

Die Umhüllung kann wahlweise als Beschichtung oder als Überzug ausgeführt sein, wobei eine Beschichtung vorzugsweise in einem abgekapselten Reaktionsraum durch isostatisches Pressen, ggf. unter zusätzlicher Anwendung einer erhöhten Temperatur erfolgt.

Die Umhüllung kann je nach Anwendungsfall oder nach Art der betreffenden Prothese Bruchteile eines Millimeters bis zu einem Millimeter dick sein und im Bedarfsfalle auch unterschiedliche Stärke an unterschiedlichen Stellen der Prothese aufweisen.

Die Umhüllung der implantierbaren Prothese kann partiell oder vollständig erfolgen.

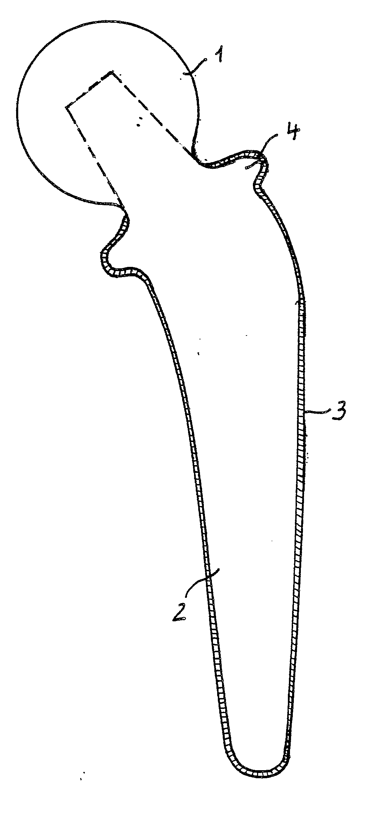

Anhand eines in der einzigen Figur der Zeichnung dargestellten Ausführungsbeispieles soll der der Erfindung zugrundeliegende Gedanke näher erläutert werden.

In der Zeichnung ist eine implantierbare Endoprothese im Längsschnitt dargestellt. Sie weist einen Prothesenkopf 1, einen Prothesenhals 4 sowie einen Prothesenschaft 2 auf, der in die entsprechend vorbereitete Markhöhle des Femurs eines Patienten eingesetzt wird. Anstelle eines Prothesenkopfes 1 kann auch eine konische Verlängerung des Prothesenhalses vorgesehen werden, auf die ein entsprechender Prothesenkopf mit einer dem Konus des Prothesenhalses angepassten konischen Ausnehmung aufgesetzt werden kann.

Im implantierten Zustand liegt der Prothesenhals 4 an der ebenen Schnittfläche des Oberschenkelknochens an.

Erfindungsgemäß ist zumindest der Prothesenschaft 2 mit einer Umhüllung aus biokompatiblem, elastischem, membranartigem Kunststoff 3 umgeben. Diese aus einem PTFE-Material bestehende Umhüllung weist eine Dicke von Bruchteilen eines Millimeters bis zu einem Millimeter auf und kann wahlweise als Beschichtung oder als Überzug ausgebildet sein.

Im Falle einer Beschichtung wird der Prothesenschaft 2 in einen abgekapselten Reaktionsraum gegeben und der biokompatible, elastische, membranartige Kunststoff mittels eines isostatischen Pressverfahrens, ggf. unter zusätzlicher Anwendung einer erhöhten Temperatur, auf den Prothesenschaft 2 aufgebracht.

Ist die Umhüllung als Überzug ausgebildet, so wird die entsprechend vorgeformte Hülle über den Prothesenschaft, der üblicherweise aus Metall, vorzugsweise Titan und/oder einer Titanlegierung besteht, bezogen und im Bereich des Prothesenhalses 4 verankert. Die Verankerung kann beispielsweise in der Weise erfolgen, daß die Hülle an dem konischen Zapfen des Prothesenhalses 4 anliegt und darananschließend der Prothesenkopf 1 mit seiner entsprechend ausgeformten konischen Ausnehmung auf den Prothesenhals 4 aufgesetzt wird, so daß zwischen Prothesenkopf 1 und Prothesenhals 4 die Hülle aus biokompatiblem, elastischem, membranartigem Kunststoff eingeklemmt wird.

Aufgrund der Elastizität ist die Umhüllung 3 in der Lage, wechselnde Beanspruchungen der implantierten Prothese zu puffern und so eine dauerhafte Verbindung zwischen der Knochenmarkhöhle und dem Prothesenschaft sicherzustellen. Der biokompatible, membranartige Kunststoff stellt darüberhinaus sicher, daß die Prothese in die vorbereitete Knochenmarkshöhle eingebracht werden kann, ohne das unerwünschte Reaktionen des die Prothese umgebenden Knochengewebes erfolgen und daß Gewebeflüssigkeiten zu einer Beeinträchtigung der Verbindung zwischen Prothesenschaft 2 und Knochenmarkhöhle führen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich andersgearteten Ausführungen Gebrauch machen.

## Ansprüche

1. Implantierbare Prothese mit einem Prothesenkopf und einem Prothesenschaft,
**dadurch gekennzeichnet, daß**
zumindest der Prothesenschaft (2) eine Umhüllung (3) aus einem biokompatiblen, elastischen, membranartigen Kunststoff aufweist.

2. Implantierbare Prothese nach Anspruch 1, **dadurch gekennzeichnet,** daß die Umhüllung (3) aus einem Polytetrafluoräthylen-Material besteht, wie es insbesondere zur Herstellung künstlicher Arterien Verwendung findet.

3. Implantierbare Prothese nach Anspruch 1 oder 2, **dadurch gekennzeichnet,** daß die Umhüllung (3) als Beschichtung ausgebildet ist.

4. Implantierbare Prothese nach Anspruch 1 oder 2, **dadurch gekennzeichnet,** daß die Umhüllung (3) als Überzug ausgebildet ist.

5. Implantierbare Prothese nach einem der vorangehenden Ansprüche, **dadurch gekennzeichnet,** daß die Umhüllung (3) eine Dicke von Bruchteilen eines Millimeters bis zu einem Millimeter aufweist.

6. Implantierbare Prothese nach Anspruch 5, **dadurch gekennzeichnet,** daß die Umhüllung (3) eine unterschiedliche Dicke an verschiedenen Stellen des Prothesenschaftes (2) aufweist.

//

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 444 831 (DOW CORNING CO.) <br> * Ansprüche 1,3; Seite 6, Absatz 2; Figuren 1,4 * | 1,4 | A 61 F 2/30 |
| Y | --- | 2,3 | |
| Y | FR-A-2 138 735 (VITEX INC.) <br> * Seite 5, Zeilen 5-14; Seite 6, Zeilen 32-36; Seite 8, Zeilen 16-29 * | 2,3 | |
| A | --- <br> DE-A-2 808 740 (OMAR-PACHA) <br> * Figur 7 * <br> ----- | 6 | |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 04-04-1989 | SANCHEZ Y SANCHEZ J. |

EPO FORM 1503 03.82 (P0403)